# EUROPÄISCHE PATENTSCHRIFT

(11) **EP 2 459 782 B1**
(45) Veröffentlichungstag und Bekanntmachung des Hinweises auf die Patenterteilung: **21.08.2013**
(21) Anmeldenummer: 10729916.6
(22) Anmeldetag: 09.07.2010
(51) Int. Cl.: D01D 5/247, D01F 2/06

(54) **REGENERIERTE CELLULOSEFASER**
Regenerated cellulose fibre
Fibre de cellulose régénérée

(30) Priorität: 31.07.2009 EP 09450139
(43) Veröffentlichungstag der Anmeldung: 06.06.2012
(73) Patentinhaber: Kelheim Fibres GmbH, 93309 Kelheim (DE)
(72) Erfinder: BERNT, Ingo, 93053 Regensburg (DE); ROGGENSTEIN, Walter, 93077 Bad Abbach (DE); WOODINGS, Calvin, Eathorpe Warwickshire CV33 9DF (GB); HARMS, Haio, A-4810 Gmunden (AT)
(74) Vertreter: Nemec, Harald
(86) Internationale Anmeldenummer: PCT/EP2010/059870
(87) Internationale Veröffentlichungsnummer: WO 2011/012424

(56) Entgegenhaltungen:
- GB-A- 758 651
- GB-A- 1 086 496
- GB-A- 1 333 047
- GB-A- 1 393 778
- JP-A- 53 143 722
- US-A- 2 476 293
- US-A- 3 318 990
- US-A- 3 699 965
- US-A- 4 182 735
- C.R. WOODINGS AND A.J. BARTHOLOMEW: "The Manufacture, Properties and Uses of Inflated Viscose Fibres" LENZINGER BERICHTE, Bd. 58, 1. Januar 1985 (1985-01-01), Seiten 33-39, XP002562009 ISSN: 0024-0907

## Beschreibung

Die vorliegende Erfindung betrifft eine regenerierte Cellulosefaser, die durch das Viskoscverfahren erhalten wird.

Insbesondere betrifft die Erfindung eine regenerierte Cellulosefaser mit kollabierter hohler Querschnittsstruktur.

Für Hygieneanwendungen wie zum Beispiel Tampons oder Saugkörper im Allgemeinen sind Fasern mit besonders hohem Flüssigkeitsspeichervermögen wünschenswert, um so eine möglichst hohe Absorptionskapazität des Hygieneprodukts zu ermöglichen.

Konventionelle superabsorbierende Materialien haben den Nachteil, dass sie in der Regel bei Kontakt mit Wasser ein Gel bilden und so die Integrität des Saugkörpers verringern und zudem den Flüssigkeitsweitertransport durch den Saugkörper blockieren. Die Integrität des Saugkörpers ist in diesen Anwendungen auch deshalb wichtig, weil so ein Austreten von Absorptionsmaterial und somit eine Kontamination beispielsweise von Wunden mit diesem Material verhindert wird.

Für Wundauflagen ist es ebenfalls wünschenswert, dass das absorbierende Material nicht mit der Wunde verklebt, so dass ein schmerzfreies Ablösen der Auflage von der Wunde erreichbar ist.

Eine weitere Forderung an Fasermaterial in diesen Anwendungsgebieten ist die einwandfreie Verarbeitbarkeit in konventionellen Produktionstechniken der Vliesherstellung.

Viskosefasern mit einem Anteil an Carboxymethylcellulose (CMC) sind bekannt. Es handelt sich dabei um eine Mischfaser, die durch das Einspinnen von Carboxymethylzellulose in die Viskosespinnmasse erhalten wird. Solche Fasern wurden auch kommerziell hergestellt (US 4,199,367 A, US 4,289,824 A).

Die Herstellung von Viskosefasern mit einer hohlen Querschnittsstruktur ("Hohlfasern") durch Einspinnen von Natriumcarbonat in die Viskosespinnmasse ist bereits seit 1920 bekannt. Fasern dieser Art wurden früher von unterschiedlichen Anbietern kommerziell hergestellt. Hohlfasern vom "Superinflated"-Typ mit hohen Wasserrückhaltevermögen wurden speziell für Hygiene-Anwendungen hergestellt (GB 1,333,047 A, GB 1,393,778 A).

Ein umfassende Übersicht über Entwicklung und Geschichte von Viskose-Hohlfasern findet sich unter: C.R. Woodings, A. J. Bartholomew; "The manufacture properties and uses of inflated viscose rayon fibres"; TAPPI Nonwovens Symposium; 1985; pp. 155-165.

Viskose-CMC-Mischfasern weisen in der Herstellung den Nachteil auf, dass bedingt durch ihre hohe Saugfähigkeit und das starke Aneinanderhaften der Fasern im nassen Zustand (Geleffekt an der Faseroberfläche) die Fasern ab einem Einlagerungsgrad von ca. 15% CMC bezogen auf Cellulose im herkömmlichen Viskoseverfahren dazu neigen, in den Bädern der Nachbehandlung abzusinken und nicht wie herkömmliche Viskosefasern aufzutreiben. Dieses erschwert die Herstellung dieser Fasern in einem üblichen kommerziellen Verfahren erheblich.

Ein erwünschter Effekt dieser Fasern ist ihre gelartige Oberflächenkonsistenz im feuchten Zustand. Dieser Effekt wird im Griff aber wieder teilweise dadurch verringert, dass diese Fasern genauso wie herkömmliche Viskosefasern eine stark geriffelte Oberflächenstruktur aufweisen.

Die maximale Steigerung des Wasserrückhaltevermögens solcher Fasern ist limitiert, da es bei Einspinnen von >50 % CMC zu massiven Verarbeitungsproblemen kommt.

Auch bei Viskosehohlfasern ist die Steigerung des Wasserrückhaltevermögens limitiert. Durch eine stärkere Inflation der Faser wird ab einem bestimmten Grad kein höheres Wasserrückhaltevermögen mehr erreicht, es kommt vielmehr zur Bildung von bandartigen Fasern mit flachem Querschnitt welche ein geringeres Wasserrückhaltvermögen aufweisen und deren Nachbehandlung in einem Stapelfaserverfahren, aufgrund ihrer sehr großen und glatten Oberfläche und dem damit verbundenen sehr hohen Faser-Faser Bindevermögen, äußerst problematisch ist.

Bekannt sind weiters carboxymethylierte Fasern, d.h. Fasern, die durch die nachträgliche Carboxymethylierung von Viskosefasern hergestellt werden (kommerziell erhältlich als "Aquacell"-Fasern der Fa. Convatec; US 6,548,730 B1; AU 757461 B; WO00/01425 A1 ; EP 1 091 770 A1). Diese Fasern ermöglichen zwar eine sehr hohe Wasseraufnahme allerdings bilden diese Fasern bei Kontakt mit Wasser unter vollständigem Verlust der Faserstruktur ein Gel, was nicht für alle möglichen Anwendungen wünschenswert ist.

Die US 3,318,990 beschreibt ein Verfahren zur Herstellung von Viskose-Flachfasern, bei welchem in die Viskose einerseits Natriumcarbonat und andererseits eine im Wasser quellende hochmolekulare Substanz, z.B. CMC, zugemischt werden.

Die resultierenden Fasern werden als vollkommen flach beschrieben. Die kollabierte Querschnittsstruktur hält auch im feuchten Zustand fest zusammen. Die Fasern eignen sich gemäß US 3,318,990 zur Herstellung von Papier.

Die vorliegende Erfindung stellt sich zur Aufgabe, eine Viskosefaser mit möglichst hohem Wasserrückhaltevermögen, einer möglichst hohen Absorption unter Druck und einem zusätzlichen Oberflächen-Geleffekt zur Verfügung zu stellen. Die Faser soll sich mit den üblichen Verfahren zur Viskosefaserherstellung produzieren lassen und sich nach dem üblichen Verfahren zur Vliesherstellung weiterverarbeiten lassen (beispielsweise Krempeln; Verfestigung durch Vernadlung, Wasserstrahlverfestigung, thermische Verfestigung, Kalandrieren).

Diese Aufgabe wird mit einer regenerierten Cellulosefaser gemäß Anspruch 1 gelöst. Zur Herstellung der erfindungsgemäßen Cellulosefaser dient das Verfahren gemäß Anspruch 11. Bevorzugte Ausführungsformen sind in den Unteransprüchen angeführt.

### KURZE BESCHREIBUNG DER FIGUREN

Figur 1 zeigt die Längsansicht einer getrockneten erfindungsgemäßen Cellulosefaser.
Figur 2 zeigt eine stärker vergrößerte Längsansicht einer getrockneten erfindungsgemäßen Cellulosefaser.
Figur 3 zeigt den Querschnitt von getrockneten erfindungsgemäßen Cellulosefasern.
Figur 4 zeigt den röhrenförmigen Querschnitt einer erfindungsgemäßen Cellulosefaser nach Kontakt mit Wasser.

### DETAILLIERTE BESCHREIBUNG DER ERFINDUNG

Die vorliegende Erfindung stellt eine regenerierte Cellulosefaser mit kollabierter hohler Querschnittsstruktur zur Verfügung, die durch die Kombination der folgenden Eigenschaften gekennzeichnet ist:
- die Faser weist im trockenen Zustand eine kollabierte hohle Querschnittsstruktur auf
- die Faser weist im feuchten Zustand eine Querschnittstruktur mit Hohlräumen auf
- die Faser weist in Längsrichtung eine Segmentierung durch Trennwände auf
- in die Faser ist ein saugfähiges Polymer inkorporiert.

Bevorzugt ist das saugfähige Polymer Carboxymethylcellulose (CMC). Andere geeignete saugfähigen Polymer können aus der Gruppe bestehend aus mit dem Viskoseverfahren kompatiblen Polymeren, wie z.B. Viskosecarbamat, Carboxyethylcellulose, Alginaten oder Homo- und Copolymeren von Acrylsäuren (wie zum Beispiel in US4399255 beschrieben) ausgewählt werden.

Mit der vorliegenden Erfindung wird somit in der bevorzugten Ausführungsform erstmalig eine Kombination aus einer Viskosehohlfaser, die bei Wiederbefeuchten wieder eine röhrenförmige Struktur mit Hohlräumen aufweist, und einer Viskosemischfaser mit CMC bereitgestellt.

Unter einer regenerierten Cellulosefaser mit kollabierter hohler Querschnittsstruktur versteht der Fachmann eine Viskosefaser, deren Querschnitt so stark aufgebläht ("inflated") wurde, dass die Querschnittstruktur in sich selbst kollabiert. Die Herstellung solcher Fasern (durch Beimischen von Natriumcarbonat) wird in der GB 1,333,047 A und der GB 1,393,778 A beschrieben.

Die erfindungsgemäße Faser hat im getrockneten Zustand bevorzugt einen unregelmäßigen, mehrschenkeligen oder unregelmäßig gerippten Querschnitt. Solch Fasern werden auch als "SI" (für "superinflated")-Fasern bezeichnet. Im Unterschied zu den in der US 3,318,990 A beschriebenen Fasern öffnet sich aber bei einem Befeuchten der Fasern der Querschnitt wieder und wird unter Ausbildung von Hohlräumen wieder röhrenförmig.

Die erfindungsgemäße Faser unterscheidet sich zudem insbesondere von den in der US 3,318,990 A beschriebenen Fasern durch das Vorliegen einer Segmentierung in Längsrichtung aufgrund von Trennwänden.

Der Abstand der Trennwände beträgt dabei bevorzugt zwischen 0,3 und 3 mal, bevorzugt 0,5-2 mal der über die Faserlänge gemittelten Faserbreite. Es wird vermutet, dass die Anwesenheit dieser Trennwände dafür mitverantwortlich ist, dass sich bei Befeuchten der Faser wieder eine röhrenförmige Struktur mit Hohlräumen ausbildet.

Diese röhrenförmige Struktur ist vermutlich dafür verantwortlich, dass die erfindungsgemäße Faser im Unterschied zu den auch bei Wiederbefeuchten flachen Fasern der US 3,318,990 eine hervorragende Saugfähigkeit aufweist.

Unter der Maßnahme, dass in die Faser ein saugfähiges Polymer, insbesondere CMC inkorporiert ist, versteht der Fachmann, dass in die Matrix der (nach Regeneration der Faser) underivatisierten Cellulose das saugfähige Polymer, z.B. CMC eingelagert ist. Dies ist - im Unterschied zu einem Aufbringen von CMC auf die bereits fertige Faser - insbesondere durch Einspinnnen von CMC in die Spirinviskose möglich.

Die erfindungsgemäße Cellulosefaser hat ein sehr hohes Wasserrückhaltevermögen; der Fasercharakter bleibt aber auch im nassen Zustand erhalten. Zudem zeigt sich, dass die erfindungsgemäße Faser im Unterschied zu herkömmlichen Viskose-CMC-Mischfasern in einem konventionellen Viskoseverfahren gut herstellbar und insbesondere gut verarbeitbar ist.

Der Anteil an saugfähigem Polymer, insbesondere an CMC, in der erfindungsgemäßen Faser beträgt bevorzugt 5 Gew.% bis 50 Gew.%, besonders 15 Gew.% bis 40 Gew.%, am meisten bevorzugt 20 Gew.% bis 30 Gew.%, bezogen auf underivatisierte Cellulose.

Die erfindungsgemäße Cellulosefaser liegt bevorzugt in Form einer Stapelfaser vor und kann in allen üblichen Titerbereichen hergestellt werden. Bevorzugt kann der Fasertiter von 0,5 dtex bis 8 dtex, bevorzugt von 1,3 bis 6 dtex betragen.

Die Faserlänge der erfindungsgemäßen Faser kann von 2 mm bis 80 mm betragen und hängt insbesondere vom Anwendungsgebiet ab. Für einen Wetlaidprozess sind insbesondere Faserlängen von 2 bis 20 mm geeignet, für einen Kardierprozess Faserlängen von 20 bis 80 mm.

Die Reissfestigkeit der erfindungsgemäßen Faser liegt typischerweise bei über 10cN/tex; die Reissdehnung bei über 15%.

Die erfindungsgemäße Cellulosefaser weist bevorzugt ein Wasserrückhaltevermögen von mindestens 300%, gemessen nach DIN 53814, auf.

Es wurde beispielsweise bei erfindungsgemäßen Fasern mit einem Anteil von 35% Gew.% CMC ein Wasserückhaltevermögen von bis zu 400% WRV gemessen.

Ein aus der erfindungsgemäßen Faser hergestellter zylindrischer Tampon mit einer Masse von 2,72 g, einer Länge von 44 mm und einem Durchmesser von 13 mm weist bevorzugt einen Syngina-Wert von mindestens 5,2 g/g, gemessen nach EDANA / INDA Standard Test Mcthods for the Nonwovens and Related Industries ERT 350.0 bzw. WSP 350.1, auf.

Damit weist die Faser einen höheren Syngina-Wert als die derzeit kommerziell bedeutendste saugfähige Viskosefaser, eine unter dem Markennamen "Galaxy"® vermarktete Viskosefaser mit regelmäßigem trilobalem Querschnitt (EP 0 301 874 A1), auf.

Zur Herstellung einer regenerierten Cellulosefaser gemäß der vorliegenden Erfindung dient ein Verfahren, umfassend die Schritte
- Bereitstellen einer Viskosespinnmasse
- Einmischen eines mit Viskose kompatiblen Carbonates in die Viskosespinnmasse
- Einmischen eines saugfähigen Polymers in die Viskosespinnmasse
- Verspinnen der Viskosespinnmasse in ein Spinnbad unter Bildung von Fasern,
wobei
- die Reife der Viskosespinnmasse vor dem Verspinnen weniger als 15° Hottenroth, bevorzugt 10° bis 14° Hottenroth beträgt
- der Gehalt an H₂SO₄ im Spinnbad 8% bis 10% beträgt
- der Gehalt an ZnSO₄ im Spinnbad 0,3% bis 0,5% beträgt und
- der Gehalt an Na₂SO₄ im Spinnbad 25% bis 30% beträgt.

Das erfindungsgemäße Verfahren kombiniert somit den an sich bekannten Schritt des Einspinnens eines Carbonates, insbesondere Natriumcarbonat, zur Herstellung eines hohlen Querschnittes, mit dem Schritt des Einspinnens eines saugfähigen Polymers.

Im Vergleich zu dem in der US 3,318,990 beschriebenen Verfahren wird im erfindungsgemäßen Verfahren eine Viskose mit einer geringeren Reife (angegeben in °Hottenroth) versponnen und enthält das Spinnbad einen geringeren Anteil an H₂SO₄ und ZnSO₄. Es wird vermutet, dass diese unterschiedliche Verfahrensführung zu den oben angegebenen Unterschieden zwischen der erfindungsgemäßen Faser und der in der US 3,318,990 beschriebenen Faser, insbesondere hinsichtlich der Querschnittsstruktur und auch hinsichtlich den sonstigen Eigenschaften führt.

Das saugfähige Polymer ist bevorzugt CMC. Die folgenden Ausführungcn beziehen sich auf diese bevorzugte Ausführungsform, gelten aber *mutatis mutandis* auch für andere saugfähige Polymere, die mit dem Viskoseverfahren kompatibel sind.

Als Carbonat ist jedes Carbonat, insbesondere Alkalimetallcarbonat, geeignet, welches mit den Bedingungen des Viskoseverfahrens kompatibel ist. Besonders geeignet ist Natriumcarbonat. Andere geeignete Carbonate sind Kaliumcarbonat, Kalziumcarbonat sowie ganz allgemein alle Carbonate, die unter Säureeinfluß Kohlendioxid freisetzen.

Die Beeinflussung der Querschnittsform der Faser (Aufblähen und Kollabieren) dahingehend, dass sich eine kollabierte hohle Querschnittsstruktur ergibt, z.B. durch Wahl entsprechende Bedingungen im Spinnbad, Wahl der Temperatur und natürlich Menge an zugegebenem Carbonat, ist dem Fachmann insbesondere aus den oben zitierten Patentveröffentlichungen bekannt.

Bevorzugt wird das Carbonat in einer Menge von 11 Gew.% - 23 Gew.% (gerechnet als (CO₃)²⁻). bezogen auf Cellulose in der Viskosespinnmasse, eingemischt. Im konkreten Fall von Natriumcarbonat beträgt die bevorzugte Menge von 20 Gew.% bis 40 Gew.%, bezogen auf Cellulose.

Weiters bevorzugt wird das saugfähige Polymer, insbesondere die Carboxymethylcellulose, in einer Menge von 5 Gew.% bis 50 Gew.%, besonders bevorzugt 15 Gew.% bis 40 Gew.%, am meisten bevorzugt 20 Gew.% bis 30 Gew.%, bezogen auf Cellulose in der Viskosespinnmasse, eingemischt.

Das Carbonat und/oder die Carboxymethylcellulose können bevorzugt in Form einer Lösung eingemischt werden.

Insbesondere kann die Carboxymethylcellulose in Form einer alkalischen Lösung, enthaltend 2 Gew.% bis 9 Gew.%, bevorzugt 3 Gew.% bis 5 Gew.% NaOH und 5 Gew.% bis 15 Gew.%, bevorzugt 8 Gew.%) bis 12 Gew.% Carboxymethylcellulose, eingemischt werden.

Das Carbonat und die Carboxymethylcellulose können auch gemeinsam, insbesondere in Form einer gemeinsamen Lösung, eingemischt werden.

Zur Herstellung der erfindungsgemäßen Cellulosefaser kann eine herkömmliche Viskosespinnmasse herangezogen werden.

Eine typische Ausgestaltung des erfindungsgemäßen Verfahrens umfasst folgende Maßnahmen:

Einer nach den herkömmlichen Verfahren hergestellten Viskosespinnmasse (Cellulosegehalt = 9-10%, NaOH-Gehalt 5-6%; Viskosität 30-50 Kugelfallsekunden, Reife 10-15° Hottenroth) werden 25-35 Gew.% Na₂CO₃ (je nach Fasertiter zu variieren), bezogen auf Cellulose in der Viskosespinnmasse, in Form einer 20%igen Na₂CO₃ Lösung zugesetzt. Weiter werden der Spinnmasse 5 Gew.% - 45 Gew.%, bevorzugt 20 Gew.% - 30 Gew.%, bezogen auf Cellulose in der Viskosespinnmasse, Carboxymethylcellulose in Form einer alkalischen Lösung (2-9 Gew.%) NaOH; bevorzugt 3-5 Gew.%) zugegeben. Die Konzentration der Lösung beträgt 5 Gew.% bis 15 Gew.%, bevorzugt 8-12 Gew.%. Die Carboxymethylcellulose ist ein handelsübliches Produkt mit einem Substitutionsgrad DS von 0,6 - 1,2, bevorzugt 0,65 - 0,85 und einer Viskosität (2%ige Lösung; 25°C) von 30-800 mPas; bevorzugt 50-100 mPas.

Die Additiv-Lösungen (Carbonat und CMC) werden bevorzugt in die bereits spinnreife Viskosespinnmase, insbesondere unmittelbar vor dem Verspinnen, injiziert. DieViskose wird anschließend homogenisiert, bevorzugt über einen dynamischen Mischer.

Die Viskose wird mit für textile Fasern üblichen Parametern ausgesponnen und nachbehandelt.

### Beispiele:

Zur im Folgenden beschriebenen Herstellung von regenerierten Cellulosefasern wurde eine herkömmliche Viskosespinnmasse herangezogen.
Versuch A: Verspinnen der Viskosespinnmasse ohne Additive
Versuch B: Verspinnen der Viskosespinnmasse mit 30% Na₂CO₃ bezogen auf Cellulose
Versuch C (erfindungsgemäß): Verspinnen der Viskosespinnmasse mit 30%) Na₂CO₃ und 20%) CMC (DS=0,81; Viskosität 2% = 53 mPas, Type Blanose 7M1F, Hersteller: Fa. Aqualon France (Hercules)), jeweils bezogen auf die in der Viskosespinnmasse vorhandene Cellulose.

Die Spinnmassen wurden mit identischen Spinnparametern zu Fasern mit einem Titer von 4,4 dtex ausgesponnen.
Versuch D: Verspinnen der Viskosespinnmasse ohne Additive
Versuch E: Verspinnen der Viskosespinnmasse mit 20% CMC (Spezifikation wie bei Versuch C) bezogen auf Cellulose
Versuch F: Verspinnen der Viskosespinnmasse mit 30% CMC (Spezifikation wie bei Versuch C) bezogen auf Cellulose

Die Spinnmassen wurden mit identischen Spinnparametern zu Fasern mit einem Titer von 3 dtex ausgesponnen.

### Morphologie der hergestellten Fasern

Bei mikroskopischer Betrachtung handelt es sich bei den Fasern B und C um Hohlfasern vom SI-Typ (superinflated, segmentiert). Bei den Fasern A, D, E und F handelt es sich um Fasern mit dem für Viskosefasern üblichen gerippt-runden Querschnitt.

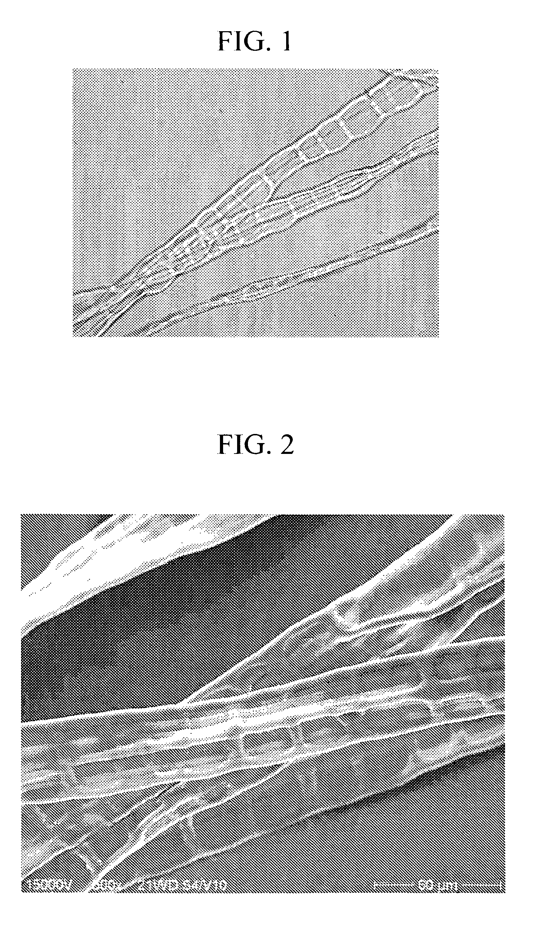

Längsansicht und Querschnitt der getrockneten erfindungsgemäßen Faser C sind in Figuren 1, 2 und 2 dargestellt.

In Figur 1 und 2 ist deutlich die für die erfindungsgemäße Faser typische Segmentierung in Längsrichtung zu sehen. Die Segmentierung wird durch Trennwände bewirkt, die - was in Figur 2 unter stärkerer Vergrößerung feststellbar ist - tatsächlich eine feste und membranartige Unterteilung bewirken.

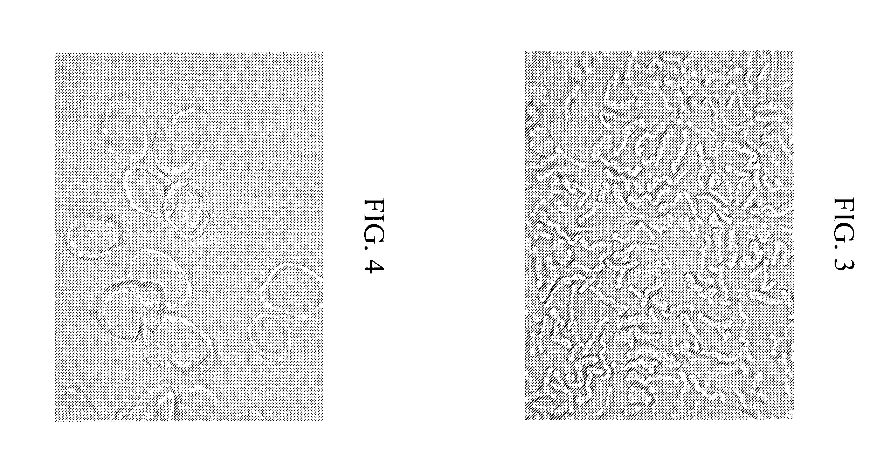

In Figur 3 ist die für "SI"-Fasern typische, kollabierte hohle Querschnittsstruktur unter Ausbildung mehrschenkeliger bzw. unregelmäßig gerippter Querschnitte erkennbar.

Figur 4 zeigt den röhrenförmigen Querschnitt der erfindungsgemäßen Faser nach einem Befeuchten der trockenen Faser.

### Spinnverhalten:

Alle Fasern lassen sich mit den üblichen Parametern verspinnen. Während die Fasern E und F in den Bädern der Fasernachbehandlung zum Absinken neigen, ist dieser Effekt bei der erfindungsgemäßen Faser C nicht zu beobachten.

### Wasserrückhaltevermögen:

Für alle Fasern wurde das Wasserrückhattevermögen in % nach DIN 53814 ermittelt.

| | |
|---|---|
| Faser A: | 80% |
| Faser B: | 220% |
| Faser C: | 335% |
| Faser D: | 93% |
| Faser E: | 149% |
| Faser F: | 193% |

Wie die Resultate zeigen, lässt sich das Wasserrücklialtevermögen, absolut betrachtet, durch Einspinnen von 20% CMC bezogen auf Cellulose um etwa 50%, durch Einspinnen von 30% CMC um 100% steigern. (Vergleich der Fasern D -> E -> F).

Durch Einspinnen von 30% Na₂CO₃ bezogen auf Cellulose lässt sich das Wasserrückhaltvermögen um 140% steigern. (Vergleich der Faser A -> B).

Durch Einspinnen von 20% CMC und 30% Na₂CO₃ bezogen auf Cellulose (erfindungsgemäß) lässt sich das Wasserückhaltvermögen dagegen um 255% steigern. Das zeigt, dass sich die Effekte der beiden Additive nicht additiv sondern eher synergistisch verstärken:

Der Effekt der Zugabe von CMC alleine bzw. Na₂CO₃ alleine beträgt:
A->B = +140%
D ->E = +56%
Erwartungswert für die Faser C wäre somit 80 + 140 + 56 = 276%

Da das gemessene Wasserrückhaltevermögen für die Faser C mit 335% um den Faktor 1,2 höher liegt, wirken die beiden Additiv-Komponenten somit in überraschender Weise synergistisch.

### Geleffekt:

Der Oberflächen-Geleffekt (schleimiger, seifiger Griff) ist bei der erfindungsgemäßen Fasern C etwas stärker ausgebildet als beim Vergleichsmuster E (bei gleichem CMC-Gehalt). Dieser Geleffekt entsteht nur im feuchten Zustand.

### Faserwerte:

Die erfindungsgemäße Faser C erreicht bei einem Titer von 4,4 dtex eine Faserfestigkeit von 13,1 cN/tex bei einer Dehnung von 22,9%, was für einen Einsatz in der Vliesverarbeitung akzeptabel ist.

### Absorption unter Druck:

Aus den Fasern A, B und C wurden Tamponplugs zu je 2,72g gepresst und eine Syngina-Messung nach WSP 351.0 durchgeführt. Als zusätzliche Referenz wurde ein identisch hergestellter Plug aus 100% kommerzieller trilobaler Viskosefaser ("Galaxy"®) hergestellt.

### Resultat:

| | |
|---|---|
| Faser A: | 4,56 g/g |
| Faser B: | 4,75 g/g |
| Faser C: | 5,73 g/g |
| 100% Galaxy: | 5,05 g/g |

Das Ergebnis zeigt, dass sich mit den erfindungsgemäßen Fasern eine deutlich erhöhte Syngina-Absorption sogar gegenüber kommerziellen trilobalen Fasern erreichen lässt.

Die erfindungsgemäße regenerierte Cellulosefaser eignet sich daher hervorragend zur Verwendung in absorbierenden Produkten, Hygieneartikeln, insbesondere Tampons, Inkontinenzprodukten, Hygienebinden und Pantylinern, Verpackungen für Lebensmitteln, insbesondere für Fleischprodukte, Papieren, insbesondere Filterpapieren, Bekleidung (z.B. Bekleidungstextilien für das Feuchtemanagement in Mischung mit anderen Fasern oder als mehrschichtige Konstruktion) und Wundauflagen.

## Patentansprüche

1. Regenerierte Cellulosefaser, **gekennzeichnet durch** die Kombination der folgenden Eigenschaften:
- die Faser weist im trockenen Zustand eine kollabierte hohle Querschnittsstruktur auf
- die Faser weist im feuchten Zustand eine Querschnittstruktur mit Hohlräumen auf
- die Faser weist in Längsrichtung eine Segmentierung **durch** Trennwände auf
- in die Faser ist ein saugfähiges Polymer inkorporiert.

2. Cellulosefaser gemäß Anspruch 1, **dadurch gekennzeichnet, dass** der Querschnitt der Faser im trockenen Zustand mehrschenkelig und/oder unregelmäßig gerippt ausgeformt ist.

3. Cellulosefaser gemäß Anspruch 1 oder 2, **dadurch gekennzeichnet, dass** der Abstand der Trennwände zwischen 0,3 und 3 mal, bevorzugt 0,5-2 mal der über die Faserlänge gemittelten Faserbreite beträgt.

4. Cellulosefaser gemäß einem der vorhergehenden Ansprüche, **dadurch gekennzeichnet, dass** der Anteil an saugfähigem Polymer in der Faser 5 Gew.% bis 50 Gew.%, bevorzugt 15 Gew.% bis 40 Gew.%, besonders bevorzugt 20 Gew.% bis 30 Gew.%, bezogen auf underivatisierte Cellulose, beträgt.

5. Cellulosefaser gemäß einem der vorhergehenden Ansprüche, **dadurch gekennzeichnet, dass** das saugfähige Polymer Carboxymethylcellulose ist.

6. Cellulosefaser gemäß einem der vorhergehenden Ansprüche, **dadurch gekennzeichnet, dass** sie in Form einer Stapelfaser vorliegt.

7. Cellulosefaser gemäß einem der vorhergehenden Ansprüche, **dadurch gekennzeichnet, dass** der Fasertiter von 0,5 dtex bis 8 dtex, bevorzugt von 1,3 bis 6 dtex beträgt.

8. Cellulosefaser gemäß einem der vorhergehenden Ansprüche, **dadurch gekennzeichnet, dass** die Faserlänge von 2 mm bis 80 mm beträgt.

9. Cellulosefaser gemäß einem der vorhergehenden Ansprüche, **gekennzeichnet durch** ein Wasserrückhaltevermögen (DIN 53814) von mindestens 300%.

10. Cellulosefaser gemäß einem der vorhergehenden Ansprüche, **dadurch gekennzeichnet, dass** ein aus der Faser hergestellter zylindrischer Tampon mit einer Masse von 2,72 g, einer Länge von 44 mm und einem Durchmesser von 13 mm einen Syngina-Wert von mindestens 5,2 g/g aufweist (EDANA ERT 350.0 oder INDA WSP 350.1).

11. Verfahren zur Herstellung einer regenerierten Cellulosefaser gemäß einem der vorhergehenden Ansprüche, umfassend die Schritte
- Bereitstellen einer Viskosespinnmasse
- Einmischen eines mit Viskose kompatiblen Carbonates in die Viskosespinnmasse
- Einmischen eines saugfähigen Polymers in die Viskosespinnmasse
- Verspinnen der Viskosespinnmasse in ein Spinnbad unter Bildung von Fasern,
wobei
- die Reife der Viskosespinnmasse vor dem Verspinnen weniger als 15° Hottenroth, bevorzugt 10° bis 14° Hottenroth beträgt
- der Gehalt an H₂SO₄ im Spinnbad 8% bis 10% beträgt
- der Gehalt an ZnSO₄ im Spinnbad 0,3% bis 0,5% beträgt und
- der Gehalt an Na₂SO₄ im Spinnbad 25% bis 30% beträgt.

12. Verfahren gemäß Anspruch 11, **dadurch gekennzeichnet, dass** das Carbonat in einer Menge von 11 Gew.% - 23 Gew.% (gerechnet als (CO₃)²⁻), bezogen auf Cellulose in der Viskosespinnmasse, eingemischt wird.

13. Verfahren gemäß Anspruch 11 oder 12, **dadurch gekennzeichnet, dass** das saugfähige Polymer in einer Menge von 5 Gew.% bis 50 Gew.%, bevorzugt 15 Gew.% bis 40 Gew.%, besonders bevorzugt 20 Gew.% bis 30 Gew.%, bezogen auf Cellulose in der Viskosespinnmasse, eingemischt wird.

14. Verfahren gemäß einem der Ansprüche 11 bis 13, **dadurch gekennzeichnet, dass** das Carbonat und/oder das saugfähige Polymer in Form einer Lösung eingemischt wird/werden.

15. Verfahren gemäß einem der Ansprüche 11 bis 14, **dadurch gekennzeichnet, dass** das saugfähige Polymer Carboxymethylcellulose ist.

16. Verfahren gemäß Anspruch 15, **dadurch gekennzeichnet, dass** die Carboxymethylcellulose in Form einer alkalischen Lösung, enthaltend 2 Gew.% bis 9 Gew.%, bevorzugt 3 Gew.% bis 5 Gew.% NaOH und 5 Gew.% bis 15 Gew.%, bevorzugt 8 Gew.% bis 12 Gew.% Carboxymethylcellulose, eingemischt wird.

17. Verfahren gemäß einem der Ansprüche 15 oder 16, **dadurch gekennzeichnet, dass** das Carbonat und die Carboxymethylcellulose gemeinsam, insbesondere in Form einer gemeinsamen Lösung, eingemischt werden.

18. Verwendung einer regenerierten Cellulosefaser gemäß einem der Ansprüche 1 bis 10 in absorbierenden Produkten, Hygieneartikeln, insbesondere Tampons, Inkontinenzprodukten, Hygienebinden und Pantylinern, Verpackungen für Lebensmitteln, insbesondere für Fleischprodukte, Papieren, insbesondere Filterpapieren, Bekleidung und Wundauflagen.

## Claims

1. A regenerated cellulose fibre, which is **characterized by** the combination of the following features:
- the fibre has in its dry condition a collapsed hollow cross-sectional structure
- the fibre has in its wet condition a cross-sectional structure with cavities
- the fibre is segmented in the longitudinal direction by dividing walls
- there is incorporated into the fibre an absorbent polymer.

2. A cellulose fibre according to claim 1, **characterized in that** the cross-section of the fibre in the dry condition is multilobal and/or is irregularly grooved.

3. A cellulose fibre according to claim 1 or claim 2, **characterized in that** the distance between the dividing walls is between 0.3 and 3 times, preferably 0.5 to 2 times the fibre width averaged over the fibre length.

4. A cellulose fibre according to any of the preceding claims, **characterized in that** the portion of absorbent polymer in the fibre is 5 % by weight to 50 % by weight, preferably 15 % by weight to 40 % by weight, especially preferably 20 % by weight to 30 % by weight, based on underivatised cellulose.

5. A cellulose fibre according to any of the preceding claims, **characterized in that** the absorbent polymer is carboxymethylcellulose.

6. A cellulose fibre according to any of the preceding claims, **characterized in that** it is present in the form of a staple fibre.

7. A cellulose fibre according to any of the preceding claims, **characterized in that** the fibre titre is 0.5 dtex to 8 dtex, preferably from 1.3 to 6 dtex.

8. A cellulose fibre according to any of the preceding claims, **characterized in that** the fibre length is from 2 mm to 80 mm.

9. A cellulose fibre according to any of the preceding claims, **characterized by** a water retention capacity (DIN 53814) of at least 300%.

10. A cellulose fibre according to any of the preceding claims, **characterized in that** a cylindrical tampon produced from the fibre with a mass of 2.72 g, a length of 44 mm and a diameter of 13 mm has a Syngina value (EDANA ERT 350.0 or INDA WSP 350.1) of at least 5.2 g/g.

11. A process for the production of a regenerated cellulose fibre according to any of the preceding claims, comprising the steps
- providing a viscose dope
- admixing a carbonate compatible with viscose into the viscose dope
- admixing an absorbent polymer into the viscose dope
- spinning the viscose dope in a spinning bath, thereby forming fibres,
wherein
- the degree of ripening of the viscose dope before spinning is less than 15° Hottenroth, preferably 10° to 14° Hottenroth,
- the content of H₂SO₄ in the spinning bath is 8% to 10%
- the content of ZnSO₄ in the spinning bath is 0.3% to 0.5% and
- the content of Na₂SO₄ in the spinning bath is 25% to 30%.

12. A process according to claim 11, **characterized in that** the carbonate is admixed in an amount of 11 % by weight to 23 % by weight (calculated as (CO₃)²⁻), based on the cellulose in the viscose dope.

13. A process according to claim 11 or 12, **characterized in that** the absorbent polymer is admixed in an amount of 5 % by weight to 50 % by weight, preferably 15 % by weight to 40 % by weight, especially preferably 20 % by weight to 30 % by weight, based on the cellulose in the viscose dope.

14. A process according to any of claims 11 to 13, **characterized in that** the carbonate and/or the absorbent polymer is/are admixed in the form of a solution.

15. A process according to any of claims 11 to 14, **characterized in that** the absorbent polymer is carboxymethylcellulose.

16. A process according to claim 15, **characterized in that** the carboxymethylcellulose is admixed in the form of an alkaline solution, containing 2 % by weight to 9 % by weight, preferably 3 % by weight to 5 % by weight NaOH and 5% by weight to 15 % by weight, preferably 8 % by weight to 12 % by weight carboxymethylcellulose.

17. A process according to any of claims 15 or 16, **characterized in that** the carbonate and the carboxymethylcellulose are admixed together, in particular in the form of a joint solution.

18. A use of a regenerated cellulose fibre according to any of claims 1 to 10 in absorbing products, sanitary products, in particular tampons, sanitary aids for incontinence, sanitary napkins and panty liners, packaging of foodstuff, in particular for meat products, papers, in particular filter papers, clothes and wound dressings.

## Revendications

1. Fibre de cellulose régénérée, **caractérisée par** la combinaison des propriétés suivantes :
- la fibre présente à l'état sec une structure transversale creuse affaissée
- la fibre présente à l'état humide une structure transversale comportant des espaces creux
- la fibre présente dans le sens longitudinal une segmentation par des parois de séparation
- dans la fibre est incorporé un polymère absorbant.

2. Fibre de cellulose selon la revendication 1, **caractérisée en ce que** la section de la fibre à l'état sec est à plusieurs branches et/ou est formée de façon irrégulièrement striée.

3. Fibre de cellulose selon la revendication 1 ou 2, **caractérisée en ce que** l'intervalle des parois de séparation se situe entre 0,3 et 3 fois, de préférence, 0,5 à 2 fois la moyenne de largeur de fibre selon la longueur de fibre.

4. Fibre de cellulose selon l'une des revendications précédentes, **caractérisée en ce que** la proportion de polymère absorbant dans la fibre est de 5 % en poids à 50 % en poids, de préférence, de 15 % en poids à 40 % en poids, de manière particulièrement préférée, de 20 % en poids à 30 % en poids, par rapport à la cellulose non dérivatisée.

5. Fibre de cellulose selon l'une des revendications précédentes, **caractérisée en ce que** le polymère absorbant est la carboxyméthylcellulose.

6. Fibre de cellulose selon l'une des revendications précédentes, **caractérisée en ce qu'**elle se présente sous la forme d'une fibre discontinue.

7. Fibre de cellulose selon l'une des revendications précédentes, **caractérisée en ce que** le titre de fibre est de 0,5 dtex à 8 dtex, de préférence, de 1,3 à 6 dtex.

8. Fibre de cellulose selon l'une des revendications précédentes, **caractérisée en ce que** la longueur de fibre est de 2 mm à 80 mm.

9. Fibre de cellulose selon l'une des revendications précédentes, **caractérisée par** une capacité de rétention d'eau (DIN 53814) d'au moins 300 %.

10. Fibre de cellulose selon l'une des revendications précédentes, **caractérisée en ce qu'**un tampon cylindrique produit à partir de la fibre présente, avec une masse de 2,72 g, une longueur de 44 mm et un diamètre de 13 mm, une valeur Syngina d'au moins 5,2 g/g (EDANA ERT 350,0 ou INDA WSP 350.1).

11. Procédé de production d'une fibre de cellulose régénérée selon l'une des revendications précédentes, comprenant les étapes de
- mise à disposition d'une masse de filage de viscose
- mélange d'un carbonate compatible avec la viscose, dans la masse de filage de viscose
- mélange d'un polymère absorbant dans la masse de filage de viscose
- filage de la masse de filage de viscose dans un bain de filage en formant des fibres,
dans lequel
- la maturation de la masse de filage de viscose avant le filage est inférieure à 15° Hottenroth, de préférence, est de 10° à 14° Hottenroth
- la teneur en H₂SO₄ dans le bain de filage est de 8 % à 10 %
- la teneur en ZnSO₄ dans le bain de filage est de 0,3 % à 0,5 % et
- la teneur en Na₂SO₄ dans le bain de filage est de 25 % à 30 %.

12. Procédé selon la revendication 11, **caractérisé en ce que** le carbonate est mélangé en une quantité de 11 % en poids à 23 % en poids (calculé en tant que (CO₃)²⁻) par rapport à la cellulose dans la masse de filage de viscose.

13. Procédé selon la revendication 11 ou 12, **caractérisé en ce que** le polymère absorbant est mélangé en une quantité de 5 % en poids à 50 % en poids, de préférence, de 15 % en poids à 40 % en poids, de manière particulièrement préférée, de 20 % en poids à 30 % en poids par rapport à la cellulose dans la masse de filage de viscose.

14. Procédé selon l'une des revendications 11 à 13, **caractérisé en ce que** le carbonate et/ou le polymère absorbant est/sont mélangé(s) sous la forme d'une solution.

15. Procédé selon l'une des revendications 11 à 14, **caractérisé en ce que** le polymère absorbant est la carboxyméthylcellulose.

16. Procédé selon la revendication 15, **caractérisé en ce que** la carboxyméthylcellulose est mélangée sous la forme d'une solution alcaline, contenant 2 % en poids à 9 % en poids, de préférence, 3 % en poids à 5 % en poids de NaOH et 5 % en poids à 15 % en poids, de préférence, 8 % en poids à 12 % en poids de carboxyméthylcellulose.

17. Procédé selon l'une des revendications 15 ou 16, **caractérisé en ce que** le carbonate et la carboxyméthylcellulose sont mélangés conjointement en particulier sous la forme d'une solution commune.

18. Utilisation d'une fibre de cellulose régénérée selon l'une des revendications 1 à 10, dans des produits absorbants, des articles hygiéniques, en particulier, des tampons, des produits pour l'incontinence, des serviettes hygiéniques et des protège-slips, des emballages alimentaires, en particulier des produits de viande, des papiers, en particulier des papiers filtres, des vêtements et des pansements.
